# EUROPÄISCHE PATENTSCHRIFT

(11) **EP 2 371 417 B1**
(45) Veröffentlichungstag und Bekanntmachung des Hinweises auf die Patenterteilung: **24.07.2019**
(21) Anmeldenummer: 11156791.3
(22) Anmeldetag: 03.03.2011
(51) Int. Cl.: A61N 1/375

(54) **Elektrische Durchführung, Verfahren zur Herstellung und Verwendung einer solchen**
Electrical feedthrough, method for the production and use thereof
Conduite électrique, procédé de fabrication et d'utilisation de celle-ci

(30) Priorität: 29.03.2010 US 318407 P
(43) Veröffentlichungstag der Anmeldung: 05.10.2011
(73) Patentinhaber: BIOTRONIK SE & Co. KG, 12359 Berlin (DE)
(72) Erfinder: Teske, Josef, 96103, Hallstadt (DE)
(74) Vertreter: Galander, Marcus

(56) Entgegenhaltungen:
- WO-A1-03/073450
- DE-A1-102008 007 346
- US-A- 5 870 272
- US-A1- 2002 027 484
- US-A1- 2005 007 718
- US-A1- 2005 092 507
- US-B1- 6 414 835

## Beschreibung

Die Erfindung betrifft eine sogenannte elektrische Durchführung (den Anschlussstift-Bereich). Sie betrifft des Weiteren ein Verfahren zur Herstellung und Verwendung einer solchen.

Elektrische Durchführungen sind in der Implantologie elektrischer Implantate seit Langem bekannt. Sie dienen dazu, elektrische Kontakte isoliert vom Implantatsgehäuse vom Inneren des Implantats nach außen zu führen, ohne dass die Dichtigkeit des Implantats gefährdet wird.

In einem einfachen Aufbau besteht eine Durchführung aus einem elektrisch leitenden Flansch, der aus demselben Material wie das Implantatsgehäuse (meist Titan) besteht, einem Anschlussstift, mit Hilfe dessen der elektrische Kontakt zwischen Implantatsinnerem und Umgebung hergestellt wird. Der Anschlussstift wird innerhalb eines Isolators, der vorzugsweise aus einer Keramik oder rein aus Glaslot besteht, geführt, welcher wiederum an seiner Außenseite mit dem Flansch verbunden ist. In der Regel sind solche elektrischen Durchführungen rund ausgeführt und führen mindestens einen, meist auch mehrere Anschlussstifte (auch Mehrfachdurchführungen genannt). Geeignet sind aber auch Einfachdurchführungen, welche einen gemeinsamen Flansch aufweisen.

Probleme können auftreten, wenn die Anschlussstifte auf der im Implantatinneren liegenden Seite mit thermischen Verfahren beispielsweise an eine elektrische Schaltung angefügt werden. In diesem Fall kann es zu Hitzeschäden an der im Implantat befindlichen elektrischen Schaltung kommen, beispielsweise wenn der Anschlussstift nur mittels Hartlöt- oder Schweißverfahren angefügt werden kann. Deshalb sollte der Anschlussstift aus einem Material bestehen, welches leicht weichlötbar ist, also auch mit Weichlot leicht benetzbar ist, damit die Schaltung beim Anfügen, beispielsweise mittels Reflow-Lötens nicht beschädigt wird. Als weichlötbares Material sind beispielsweise Nickel- oder Nickel-Legierungen bekannt. Schwierigkeiten bei den genannten Anschlussstiften bestehend aus den genannten weichlötbaren Materialen bestehen bei Verbindung des Isolators, da es zur Auflösung des Isolators kommen kann bzw. die Benetzbarkeit mit Lot schlecht ist. Ein Anschlussstift beispielsweise aus Niob (Nb), Pt, Pt/Ir, FeNi, FeNiCo, FeCr, Ta, Mo, W, Cr, FeCr, V, Ti und weiteren Metallen oder deren Legierungen lässt sich vorteilig gut hermetisch mit dem Isolator verbinden, jedoch ist Niob nur schweißbar und daher nicht brauchbar, um mit einer elektrischen Schaltung elektrisch verbunden zu werden.

Die DE 10 2008 007 346 A1 offenbart einen Kontaktstift, der sich durch eine Ausnehmung und eine darin angeordnete Glasfüllung erstreckt.

Weiterhin offenbart die US2005/0007718A1 eine Durchführung aus mindestens einem leitenden Anschlussstift, einem Durchführungsfilterkondensator, der einen Durchgang aufweist, durch den sich ein oder mehrere Anschlussstifte erstrecken.

Der Erfindung liegt daher die Aufgabe zugrunde, den Aufbau einer elektrischen Durchführung so zu verbessern, dass sie einerseits weichlötbar ist und andererseits eine optimale und dauerhaft sichere hermetische Abdichtung ermöglicht.

Diese Aufgabe wird gemäß ihrem Vorrichtungsaspekt durch eine Durchführung mit den Merkmalen des Anspruchs 1 und gemäß relativ unabhängigen Verfahrensaspekten durch ein Verfahren mit den Merkmalen des Anspruchs 9 bzw. 10 gelöst. Zweckmäßige Fortbildungen des Erfindungsgedankens sind Gegenstand der abhängigen Ansprüche.

Die Erfindung schließt den wesentlichen Gedanken ein, die gattungsgemäße Durchführung hermetisch dicht auszuführen, wobei die Durchführung das Kondensatorinnere gegenüber dem Kondensatoräußeren hermetisch dicht voneinander trennt. Sie schließt weiter den Gedanken des Vorsehens eines den Anschlussstift gegenüber dem Flansch hermetisch abdichtenden Glaslot-Pfropfes ein. Der Anschlussstift ist hierbei ein Niobstift oder ein Stift aus Pt, Pt/Ir, FeNi, FeNiCo, FeCr, Ta, Mo, W, Cr, FeCr, V, Ti oder deren Legierungen, der mindestens an einem im Inneren des Implantates liegenden Abschnitt weichlötbar ausgeführt. Im Zusammenhang mit der Erfindung ist von Bedeutung, dass im Inneren des Implantates ausschließlich weichlötbare Anschlussstiftoberflächen vorliegen. Weiterhin ist vorgesehen, dass der weichlötbare Abschnitt des Anschlussstifts im Inneren des Implantats ein Nickelstift oder ein Nickelblech ist, welcher an den Anschlussstift über eine Fügestelle angefügt ist.

Eine zweckmäßige Ausführung der Erfindung sieht vor, dass der Anschlussstift in dem Bereich seiner Länge, in dem er in Kontakt mit dem Glaslot-Pfropf steht, mindestens einen Kern aus einem Material aufweist, das annähernd den gleichen oder einen kleineren thermischen Ausdehnungskoeffizienten wie das Glaslot besitzt. Hierdurch lassen sich größere unzulässig hohe mechanische Zugspannungen im Glaslot während des Herstellens und des Einsatzes der gefertigten Durchführung grundsätzlich vermeiden. Speziell kann das Material des Anschlussstifts oder seines Kerns in dem Bereich seiner Länge, in dem er in Kontakt mit dem Glaslot-Pfropf steht, Pt, Pt/Ir, FeNi, FeNiCo, FeCr, Nb, Ta, Mo, W, Cr, FeCr, V, Ti und weiteren Metallen oder deren Legierungen bestehen.

Es ist weiter vorgesehen, dass der weichlötbare Abschnitt des Anschlussstifts im Inneren des Implantats ein Nickelstift ist. Zur zweckmäßigen Verarbeitung mit dem Glaslot im Versiegelungsabschnitt der Durchführung ist eine Ausgestaltung sinnvoll, bei der ein Stift aus Pt, Pt/Ir, FeNi, FeNiCo, FeCr, Nb, Ta, Mo, W, Cr, FeCr, V, Ti und weiteren Metallen oder deren Legierungen an den Nickelstift über eine Fügestelle angefügt, insbesondere angelötet, angeschweißt, angecrimpt oder elektrisch leitend angeklebt ist. Zur Erleichterung der weiteren Prozessführung kann insbesondere der innenseitige Nickelstift einen Nailhead aufweisen, der abgeflacht, gekrümmt, mit einer angefügten Hülse oder Scheibe versehen oder in anderer Weise gestaltet sein kann. Dieser erleichtert ein anschließendes Weichlöten des Innenanschlusses, speziell auch mittels eines Reflow-Verfahrens.

Ein weiterer bevorzugter Aufbau der vorgeschlagenen Durchführung zeichnet sich dadurch aus, dass der Anschlussstift im Flansch von einem Keramikring, insbesondere aus Al₂O₃-Keramik, umgeben ist, so dass der Keramikring für den Glaslot-Pfropf beim Lötprozess einen Abschluss bildet und den Glasverlauf damit positiv beeinflusst, so dass der Glaslot-Pfropf bei der Herstellung der Durchführung mit einer höheren Ausbeute eine hermetische Dichtung erzielen kann. Eine solche kombinierte Glas-Keramik-Durchführung erhält durch die hochgradig steife Keramik-Komponente zusätzlich eine hohe mechanische Stabilität, und durch die Keramik-Komponente wird auch eine hochwertige und zuverlässige elektrische Isolationsstrecke an den benachbarten Oberflächen von Flansch und Anschlussstift realisiert. Insbesondere bietet sie dem Glaslot-Pfropf einen mechanischen Schutz gegenüber seitlichen Krafteinwirkungen (Biegekräften), die am Anschlussstift angreifen können.

Anstatt des Keramikringes kann auch eine oder mehrere Kondensatorplatte(n) vorgesehen sein, um die elektrische Durchführung so gefiltert gegen elektromagnetische Einflüsse auszuführen. Die Platte besteht aus mehreren übereinander liegenden Platten aus leitfähigem Material, die durch dielektrische Schichten getrennt sind. Die Platten sind abwechselnd mit dem Flansch und dem Anschlussstift elektrisch verbunden und bilden so zusammen mit den dielektrischen Schichten einen Filter.

Ein weiterer bevorzugter Aufbau der vorgeschlagenen Durchführung zeichnet sich dadurch aus, dass der Anschlussstift im Flansch von zwei Keramikringen, insbesondere aus Al₂O₃-Keramik, umgeben ist. Diese beiden Keramikringe schließen den Glas-Pfropf sowohl innen- als auch außenseitig in dem Raum zwischen dem Anschlussstift und der Bohrung des Aluminiumflansches ein und verhindern beim Lötprozess ein Auslaufen des Glaslot-Pfropfs aus dem Bohrungsbereich des Flansches, so dass beim Herstellprozess mit einer höheren Ausbeute eine hermetisch dichte, mechanisch stabile, elektrisch isolierende, maßhaltige, langzeitstabile, kompakte elektrische Durchführung erzeugt werden kann.

In einer weiteren Ausführung der Erfindung ist auf der der Innenseite des Gehäuses zugewandten Seite des Glaslot-Pfropfs ein den Anschlussstift umgebender Füllstoff oder eine Beschichtung vorgesehen. Speziell ist der Füllstoff oder die Beschichtung so dimensioniert und angeordnet, dass er oder sie eine Fügestelle im Anschlussstift maskiert. Dieser Füllstoff bzw. die Beschichtung sichert die Fügestelle vor Beschädigung oder Oxidation und stellt so die sichere Funktion der elektrischen Durchführung sicher. Der Füllstoff kann ebenfalls als Pfropfen, aber auch als Belag, Beschichtung, Bedampfung o. ä. vorliegen und sollte alle in Richtung des Implantatinneren zeigenden Oberflächen der elektrischen Durchführung bedecken.

Eine zweckmäßige Verfahrensführung zur Herstellung der Durchführung, die speziell den Einsatz von Anschlussstift-Materialien mit einem vom Glaslot erheblich differierenden thermischen Ausdehnungskoeffizienten erlaubt, zeichnet sich dadurch aus, dass während der Erzeugung des Glaslot-Pfropfs der Anschlussstift mittels einer Wärmesenke gekühlt wird. Dadurch, dass hierbei der Anschlussstift kühler bleibt als das Glas, lassen sich die ansonsten auftretenden thermischen Spannungen so weit kontrollieren, dass eine hermetisch dichte Verlötung gelingt. Der Wärmeeintrag in das Glaslot kann beispielsweise durch IR-Strahlung (etwa eines CO₂-Lasers) oder induktive Wärmeeinkopplung über den umgebenden Flansch o. ä. erfolgen.

Von Vorteil ist es unter diesem Aspekt im Übrigen, die Glaslotmenge gering und die wirksame dichtende Grenzfläche zwischen Glaslot und Anschlussstift klein zu halten, um die absoluten Beträge der mechanischen Spannungen zwischen dem Glaslot-Pfropf und Pin aufgrund gegenseitiger großer Unterschiede in den thermischen Ausdehnungskoeffizienten gering zu halten.

Vorteile und Zweckmäßigkeiten der Erfindung ergeben sich im Übrigen aus der nachfolgenden Beschreibung von Ausführungsbeispielen und -aspekten anhand der Figuren. Von diesen zeigen:
- Fig. 1 und 2: Längsschnittdarstellungen von Durchführungen,
- Fig. 3: Längsschnittdarstellung einer erfindungsgemäßen Durchführung,
- Fig. 4 bis 8: Längsschnittdarstellungen von Durchführungen gemäß weiteren Ausführungen der Erfindung, mit zusätzlichem Füllstoff, und
- Fig. 9 und 10: Längsschnittdarstellungen von Durchführungen gemäß Ausführungsformen der Erfindung mit einer speziellen Anschlussstiftform.

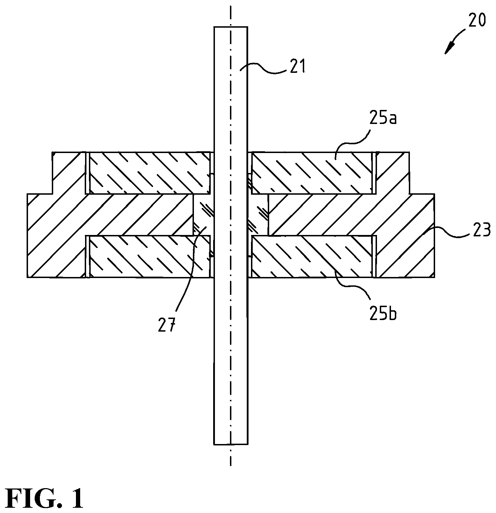

Fig. 1 zeigt eine Durchführung 20 mit einem Anschlussstift (Pin) 21, einem ober- und unterseitig mit flach zylindrischen Ausnehmungen versehenen Flansch 23 und jeweils in dem Flansch 23 liegenden Keramikscheiben 25a, 25b mit einer zentrischen Öffnung zum Durchgang des Pins 21. Außenseitig des Flansches 23 schließt sich an den Aufbau eine (hier nicht dargestellte) Gehäusewandung an, und zwischen dem Mittelteil des Al-Flansches 23 sowie den Innenwandungen der zentrischen Öffnungen der Keramikscheiben 25a, 25b sowie der Außenoberfläche des Pins 21 befindet sich ein Glaslot-Pfropf 27, der sowohl den Pin 21 als auch den Flansch 23 hermetisch gegen die umgebenden Oberflächen abdichtet, so dass sich insgesamt eine hermetische Abdichtung zwischen dem Implantatinneren und -äußeren ergibt, die zudem mit einer hohen Ausbeute mechanisch stabil, elektrisch isolierend, maßhaltig, langzeitstabil und geometrisch kompakt ist.

Als Glaslot ist ein niedrigschmelzendes Glaslot mit einer Schmelztemperatur deutlich unterhalb derer des Flanschmaterials einzusetzen, etwa ein bleihaltiges Lot des Typs G017-052 der Firma Schott mit einer Löttemperatur von ca. 410°C oder ein bleifreies Lot des G018-249, ebenfalls von Schott, mit einer Löttemperatur von 500°C. Spannungen aufgrund der voneinander abweichenden thermischen Ausdehnungskoeffizienten des Anschlussstifts und Flansches einerseits und des Glaslots andererseits können weitestgehend durch die spezielle konstruktive Gestaltung vermieden werden, die den Einsatz einer sehr geringen Glasmenge zur Erzeugung des Glaslot-Pfropfes 27 beinhaltet. Des Weiteren ist in diesem Sinne eine Kühlung des Pins 21 über eine Wärmesenke als sinnvoll anzusehen.

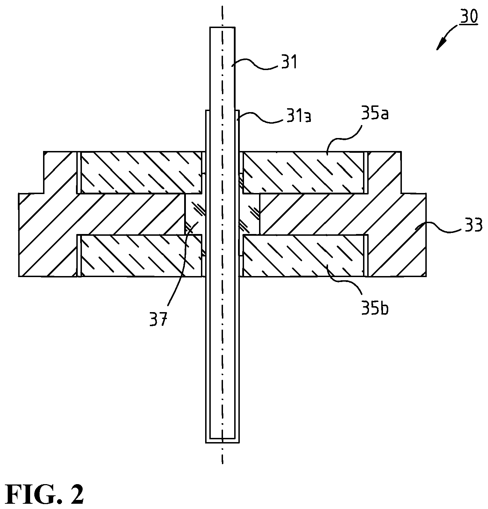

Fig. 2 zeigt einen der Ausführung nach Fig. 1 grundsätzlich ähnlichen Aufbau einer weiteren Durchführung 30. Gleiche oder ähnliche Teile sind mit an Fig. 2 angelehnten Bezugsziffern bezeichnet und werden hier nicht nochmals erläutert. Der wesentliche Unterschied gegenüber der Ausführung nach Fig. 1 besteht im Einsatz eines Pins 31 aus Platin (Pt), Pt/Ir, FeNi, FeNiCo, FeCr, Nb, Ta, Mo, W, Cr, FeCr, V, Ti oder weiteren Metallen oder deren Legierungen. Dieser Pin ist mit einem in Richtung Implantatsinneren weisenden Pinabschnitt und den innerhalb des Flansches 33 und der Keramikscheiben 35a und 35b befindlichen Abschnitt bedeckenden Ni-Beschichtung 31a. Mit diesem Aufbau des Anschlussstiftes ist dieser einerseits besser an den thermischen Ausdehnungskoeffizienten des Glases angepasst, und andererseits wird das Anbinden des Anschlussstifts an die elektrische Schaltung durch Weichlote und damit wärmeschonend möglich.

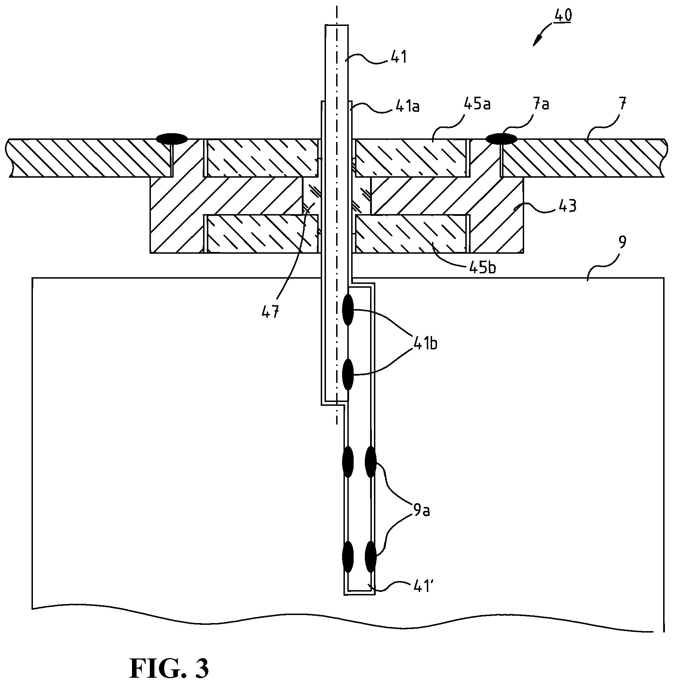

Fig. 3 zeigt eine erfindungsgemäße Durchführung 40, wo an Fig. 1 bzw. 2 angelehnte Bezugsziffern verwendet sind. Neben der eigentlichen Durchführung sind in dieser Darstellung auch die angrenzenden Abschnitte des Gehäuses 7 gezeigt, die über eine Schweißnaht 7a mit dem Flansch 43 gasdicht verbunden sind. Des Weiteren zeigt die Figur schematisch eine Schaltung 9, die sich im Inneren des Implantats befindet und wesentliche Funktionen des Implantats wie die Messung von Körpersignalen, die Abgabe von Impulsen und eventuell die drahtlose Übersendung von Daten an eine extern des Körpers befindliche Vorrichtung sicherstellt. Über ebenfalls schematisch dargestellte Füge- bzw. Schweißpunkte 9a wird die Schaltung 9 mit dem Anschlussstift verbunden.

Der erfindungsgemäße Aufbau nach Fig. 3 besteht darin, dass der Anschlussstift hier aus einem äußeren Stift 41 aus Pt, Pt/Ir, FeNi, FeNiCo, FeCr, Nb, Ta, Mo, W, Cr, FeCr, V, Ti oder weiteren Metallen oder deren Legierungen und einem inneren Stift 41' aus Ni aufgebaut ist, die über Fügestellen 41b, die z. B. durch Schweißen, Löten, Crimpen, elektrisch leitfähiges Kleben oder ein anderes, geeignetes Verfahren realisiert sind, miteinander verbunden sind. Somit ist sichergestellt, dass die Schaltung mit einem zur weichlötbaren Verbindung befähigten Material verbunden werden kann und dauerhaft verbunden bleibt.

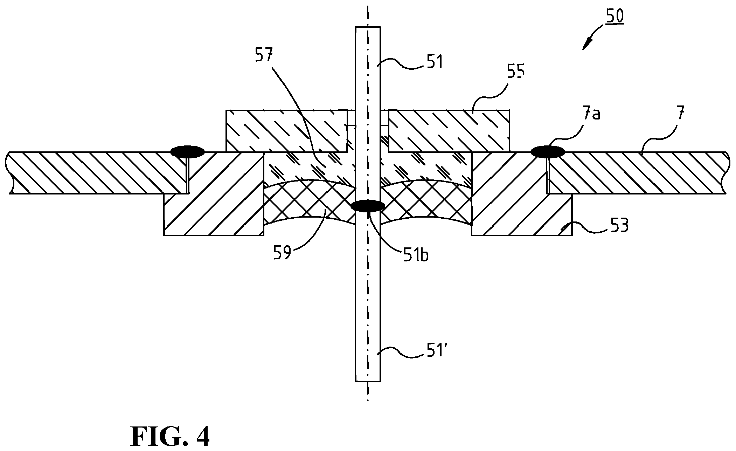

Fig. 4 zeigt eine weitere erfindungsgemäße Durchführung 50 mit einem zusammengesetzten Anschlussstift 51/51' aus einem ersten Stiftabschnitt 51 aus Pt, Pt/Ir, FeNi, FeNiCo, FeCr, Nb, Ta, Mo, W, Cr, FeCr, V, Ti oder weiteren Metallen oder deren Legierungen oder einem ähnlichen Material und einem zweiten Stiftabschnitt 51' aus Nickel. Beide Abschnitte sind durch eine Fügestelle 51b, die durch Schweißen, Löten, Crimpen, elektrisch leitfähiges Kleben oder ein anderes, geeignetes Verfahren realisiert ist, miteinander verbunden.

Weitere Unterschiede zu den vorhergehenden Ausführungen bestehen im Vorsehen eines konstruktiv einfacher aufgebauten Flansches 53 und einer einzigen Keramikscheibe 55, welche hier plan auf der Oberseite des Flansches 53 aufliegt. Die Verwendung eines Stiftabschnitts 51, dessen Wärmeausdehnungskoeffizient an den des Glaspfropfs 57 angepasst oder kleiner ist, führt dazu, dass die gemeinsame Dichtlänge größer ausfallen darf, als bei Verwendung eines herkömmlichen einteiligen Stifts und somit das Glas voluminöser ausgeführt sein darf und damit eine höhere mechanische Stabilität des Aufbaus erreicht wird. Seine dem Implantatinneren zugewandte Oberfläche sowie das aus dieser geringfügig hervorstehenden Ende des äußeren Stiftabschnitts 51 sowie die Fügestelle 51b sind in einen Füllstoff-Pfropf 59 eingebettet. Beim Füllstoff handelt es sich etwa um Gummi, eine SiliKonmasse, einem Polymer oder einen anderen halogen- bzw. chlorfreien Kunststoff. Der Füllstoff-Pfropf 59 stellt sicher, dass die Fügestelle 51b vor Beschädigung und Oxidation geschützt ist und so die Funktion des Anschlussstifts sichergestellt ist.

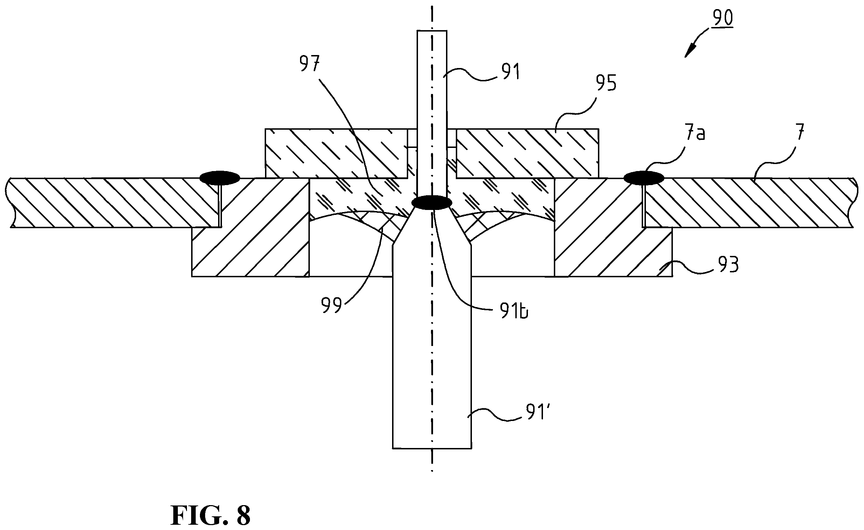

In einer weiteren Designvariante der Durchführung 90 wird wie in Fig. 8 gezeigt mindestens die Fügestelle 91b und der sich daran anschließende Teil des Stifts 91' und der Übergang zur frei liegenden Oberfläche des Glaspfropfs 97 von einem Füllstoff-Pfropf 99 überdeckt. Der Füllstoff-Pfropf 99 kann dabei auch als vergleichsweise dünne, Volumen sparende Beschichtung ausgeführt sein.

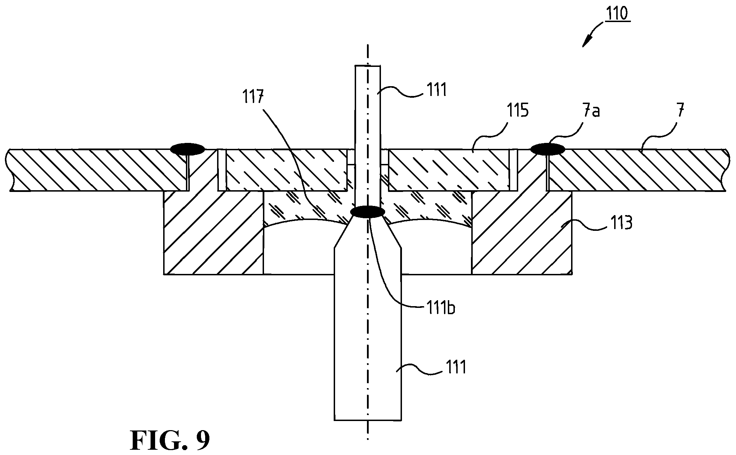

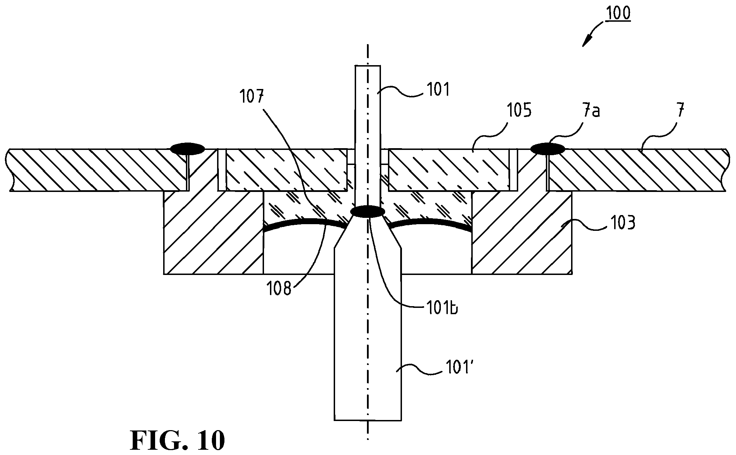

In einer weiteren Designvariante der Durchführung 110 in Fig. 9 kann die Fügestelle 111b teilweise oder vollständig und alternativ zusätzlich ein Teil des Stiftabschnitts 111' vom Glaspfropf 117 umschlossen sein. Selbst bei einem gleichzeitigen Umschließen der Fügestelle 111b und dem sich daran anschließenden Teil des Stiftabschnitts 111' durch den Glaspfropf 117 kann der Glaspfropf aufgrund der vergleichsweise großen Wärmeausdehnungskoeffizienten der Fügestelle 111b und des oder 111' im Allgemeinen keinen hermetisch dichten Verschluss dieser Teile gegenüber dem Implantatinneren sicherstellen. Diese Aufgabe sollte in einer bevorzugten Durchführungsvariante 100 wie in Fig. 10 dargestellt durch einen Füllstoff-Pfropf oder von einer entsprechenden Beschichtung 108 übernommen werden, die mindestens den Übergang vom Glaspfropf 107 zum Stift 101' bzw. zur Fügestelle 101b vollständig bedeckt und damit gegenüber dem Implantatinneren maskiert.

In einer weiteren Designvariante umschließen zwei Keramikringe den Glaspfropf von innen und außen, wobei der zweiteilige Stift durch eine Öffnung der Keramikringe geführt ist und vom Glaspfropf hermetisch dicht umschlossen wird. Die Fügestelle kann sich dabei ganz oder teilweise innerhalb des Glaspfropfs und ganz oder teilweise innerhalb des im Implantatinneren befindlichen inneren Keramikrings befinden. In einer bevorzugten Designvariante werden mindestens die Fügestelle, der Stiftabschnitt und alternativ zusätzlich auch der Glaspfropf von einem Füllstoff-Pfropf bzw. einer Beschichtung überdeckt. Dabei kann der innere Keramikring und der Flansch sowie das Gehäuse ganz oder teilweise vom dem Füllstoff-Pfropf bzw. der Beschichtung überdeckt bzw. benetzt sein.

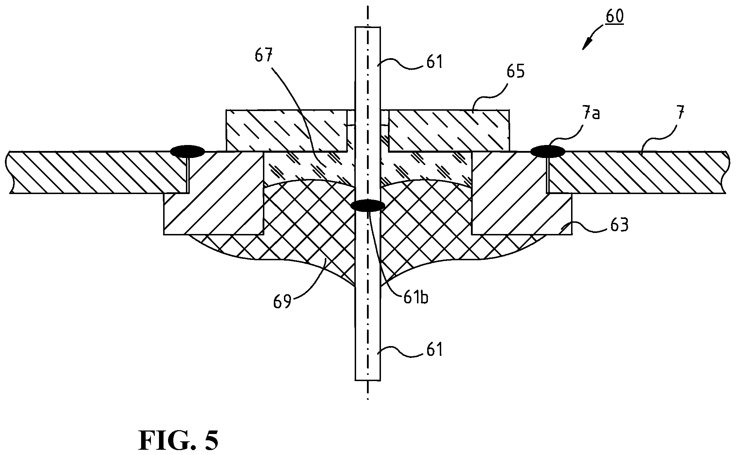

Fig. 5 zeigt eine geringfügige Abwandlung der in Fig. 4 dargestellten Konstruktion 60, bei der der Füllstoff-Pfropf 69 die Bohrung des Flansches 53 vollständig ausfüllt und einen größeren Bereich des inneren Anschlussstift-Abschnitts 61' benetzt. Dies hat den Vorteil, dass sich die Fügestelle 61b zwischen den Stiftabschnitten 61 und 61' nicht innerhalb der Bohrung des Flansches 63 befinden muss und daher leichter gebildet werden kann.

Die Fügestelle kann im Übrigen auch eine Schweiß- oder Löt- oder elektrisch leitfähige Klebeverbindung sein, oder die Stiftabschnitte können (über ein zusätzliches Element oder über einer Öffnung (Bohrung) oder eines Spalts eines der beiden Stifte) miteinander vercrimpt sein.

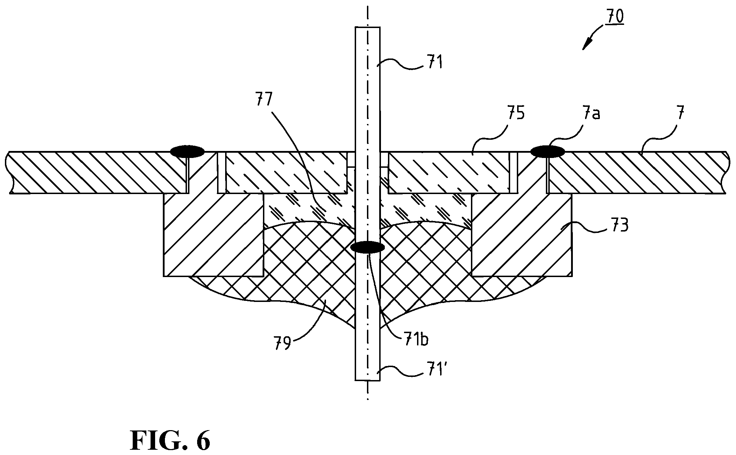

Eine weitere, in Fig. 6 gezeigte Durchführung 70 stellt eine geringfügige Abwandlung der in Fig. 5 gezeigten Ausführung dar, bei der in der Oberseite des Flansches 73 eine erweiterte Ausnehmung vorgesehen und in diese die Keramikscheibe 75 derart eingefügt ist, dass ihre obere Stirnfläche in etwa bündig mit der oberen Stirnfläche des Flansches 73 abschließt und somit insgesamt (unter Einbeziehung des oberen Gehäuseabschnitts 7) eine im Wesentlichen ebene Oberfläche der Durchführung und der zugehörigen Stirnfläche des Implantatgehäuses geschaffen wird. In einer hier nicht gezeigten Designvariante kann dieses Prinzip mit der eingelassenen Keramikscheibe auch auf eine auf der Innenseite Implantatgehäuses befindliche Keramikscheibe übertragen sein. Die mit einer zentralen Öffnung versehene(n) Keramikscheibe(n) übernimmt (übernehmen) dabei die Aufgabe der Zentrierung des Stiftes gegenüber der zentralen Flanschöffnung während der Verlötung der Glas/Keramik-Durchführung im Herstellungsprozess.

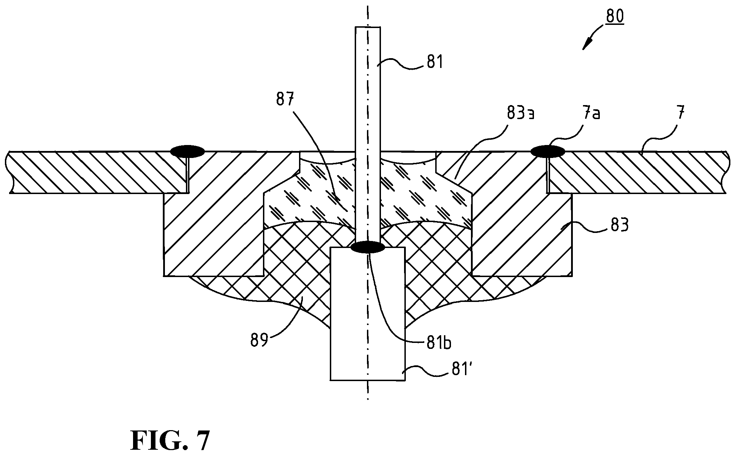

Bei der in Fig. 7 gezeigten weiteren Durchführung 80 hat der Flansch 83 eine weiter modifizierte Gestalt, die einen Fortfall der bei den anderen Ausführungen vorgesehenen Keramikscheibe(n) ermöglicht. Um für einen günstigen Glaslotverlauf und eine günstige Position des Glaslot-Pfropfens 87 im Flansch 83 zu sorgen, hat dieser hier einen nach innen weisenden angeschrägten Abschnitt 83a, der der während des Herstellprozesses den Glaslot-Pfropf positioniert und in der Anwendung bis zu einem gewissen Grade die mechanische Funktion der Keramikscheibe übernimmt. Der innere Abschnitt 81' des Anschlussstiftes ist als Designvariante hier breiter (bzw. dicker) als der äußere Stiftabschnitt 81, in dessen Bereich die hermetisch dichte Versiegelung über das Glaslot hergestellt wird. Es kann sich bei dem inneren Abschnitt übrigens auch um ein Blech o. ä. handeln.

Der angeschrägte Abschnitt 83a kann in einer weiteren Designvariante wegfallen und der Flansch 73 somit eine stufenlose, zylinderförmige Öffnung besitzen, wobei dann durch entsprechende Maßnahmen während des Herstellungsprozesses wie z. B. des Einsatzes von vom Glaslot-Pfropf 87 nicht benetzbaren Zentrierungen wie Graphit die Position des Glaslot-Pfropfes 87 in der Öffnung des Flansches 83 festgelegt wird.

Die Ausführung der Erfindung ist nicht auf die oben beschriebenen Beispiele und hervorgehobenen Aspekte beschränkt, sondern ebenso in zahlreichen Abwandlungen möglich, die im Rahmen fachgemäßen Handelns liegen.

## Patentansprüche

1. Durchführung (40, 50, 60, 70, 80, 90, 100, 110) in einem medizinelektronischen Implantat, mit einem Anschlussstift (41, 51, 61, 71, 81, 91, 101, 111), wobei der Anschlussstift ein Niobstift oder ein Stift aus Pt, Pt/Ir, FeNi, FeNiCo, FeCr, Ta, Mo, W, Cr, FeCr, V, Ti oder deren Legierungen ist, einem den Anschlussstift umgebenden Flansch (43, 53, 63, 73, 83, 93, 103, 113) und einem den Anschlussstift gegenüber dem Flansch hermetisch abdichtenden Glaslot-Pfropf (47, 57, 67, 77, 87, 97, 107, 117), **dadurch gekennzeichnet, dass** der Anschlussstift mindestens im Inneren des Implantates einen weichlötbaren Abschnitt (41', 51', 61', 71', 81', 91', 101', 111') aufweist, der Nickel oder Nickel-Legierungen umfasst, wobei der weichlötbare Abschnitt des Anschlussstifts im Inneren des Implantats ein Nickelstift oder ein Nickelblech ist, wobei der Nickelstift oder das Nickelblech an den Anschlussstift über eine Fügestelle angefügt ist.

2. Durchführung nach Anspruch 1, **dadurch gekennzeichnet, dass** der Anschlussstift (41, 51, 61, 71, 81, 91, 101, 111) in dem Bereich seiner Länge, in dem er in Kontakt mit dem Glaslot-Pfropf (47, 57, 67, 77, 87, 97, 107, 117) steht, mindestens einen Kern aus einem Material aufweist, das annähernd den gleichen thermischen Ausdehnungskoeffizienten wie das Glaslot hat.

3. Durchführung nach Anspruch 2, **dadurch gekennzeichnet, dass** das Material des Anschlussstifts (41, 51, 61, 71, 81, 91, 101, 111) oder seines Kerns in dem Bereich seiner Länge, in dem er in Kontakt mit dem Glaslot-Pfropf (47, 57, 67, 77, 87, 97, 107, 117) steht, Pt, Pt/Ir, FeNi, FeNiCo, FeCr, Nb oder Ta ist.

4. Durchführung nach einem der vorhergehenden Ansprüche, **dadurch gekennzeichnet, dass** der Anschlussstift ein Niobstift ist, welcher an den Nickelstift oder das Nickelblech (51', 61', 71', 81', 91', 101', 111') über die Fügestelle (41b, 51b, 61b, 71b, 81b, 91b, 101b, 111b) angelötet, angeschweißt, angecrimpt oder elektrisch leitend angeklebt ist.

5. Durchführung nach einem der vorhergehenden Ansprüche, **dadurch gekennzeichnet, dass** der innenseitige Nickelstift (51', 61', 71') einen Nailhead aufweist.

6. Durchführung nach einem der vorangehenden Ansprüche, **dadurch gekennzeichnet, dass** der Anschlussstift (41, 51, 61, 71, 81, 91, 101, 111) im Flansch (43, 53, 63, 73, 83, 93, 103, 113) von einem Keramikring (45a 45b, 55, 65, 75, 95, 105, 115), insbesondere aus Al₂O₃-Keramik, umgeben und der Glaslotpfropf (47, 57, 67, 77, 87, 97, 107, 117) so ausgeführt ist, dass er einen Ringspalt zwischen dem Anschlussstift und dem Keramikring hermetisch dicht verschließt.

7. Durchführung nach einem der vorangehenden Ansprüche, **dadurch gekennzeichnet, dass** innenseitig des Glaslot-Pfropfs (57, 67, 77, 87, 97, 107, 117) ein den Anschlussstift (51, 61, 71, 81, 91, 101, 111) umgebender Füllstoff (59, 69, 79, 89, 99, 108) vorgesehen ist.

8. Durchführung nach Anspruch 7, **dadurch gekennzeichnet, dass** der Füllstoff (59, 69, 79, 89, 99, 108) so dimensioniert und angeordnet ist, dass er die Fügestelle (51b, 61b, 71b, 81b) im Anschlussstift (51, 61, 71, 81) maskiert.

9. Verfahren zur Herstellung einer Durchführung nach einem der Ansprüche 1 bis 8, **dadurch gekennzeichnet, dass** während der Erzeugung des Glaslot-Pfropfs (47, 57, 67, 77, 87, 97, 107, 117) der Anschlussstift ( 41, 51, 61, 71, 81, 91, 101, 111) mittels einer Wärmesenke gekühlt wird.

10. Verwendung einer Durchführung nach einem der Ansprüche 1 bis 8, **dadurch gekennzeichnet, dass** ein innenseitiges Weichlöten am Anschlussstift (41, 51, 61, 71, 81, 91, 101, 111) mittels eines Reflow-Verfahrens ausgeführt wird.

## Claims

1. A feedthrough (40, 50, 60, 70 80, 90, 100, 110) in an electronic medical implant, having a connector pin (41, 51 ,61, 71, 81, 91, 101, 111), wherein the connector pin is a niobium pin or a pin made of Pt, Pt/Ir, FeNi, FeNiCo, FeCr, Ta, Mo, W, Cr, FeCr, V, Ti, or alloys thereof, having a flange (53, 63, 73, 83, 93, 103, 113) surrounding the connector pin and a glass solder plug (57, 67, 77, 87, 97, 107, 117) hermetically sealing the connector pin from the flange, **characterized in that** at least in the interior of the implant the connector pin has a soft solderable segment (41', 51', 61', 71', 81', 91', 101', 111') that comprises nickel or nickel alloys, wherein the soft solderable segment of the connector pin in the interior of the implant is a nickel pin or a nickel sheet, wherein the nickel pin or the nickel sheet is joined to the connector pin via a joint.

2. The feedthrough according to claim 1, **characterized in that** the connector pin (41, 51,61, 71, 81, 91, 101 111) has, in the region of its length, in which region it is in contact with the glass solder plug (47, 57, 67, 77, 87, 97, 107, 117), at least one core made of a material that has approximately the same thermal expansion coefficient as the glass solder.

3. The feedthrough according to claim 2, **characterized in that** the material of the connector pin (41, 51, 61, 71, 81, 91, 101, 111) or its core in the region of its length, in which region it is in contact with the glass solder plug (47, 57, 67, 77, 87, 97, 107, 117), is Pt, Pt/Ir, FeNi, FeNiCo, FeCr, Nb, or Ta.

4. The feedthrough according to any of the foregoing claims, **characterized in that** the connector pin is a niobium pin that is soldered, welded, crimped or glued in an electrically conducting manner to the nickel pin or to the nickel sheet (51', 61', 71', 81', 91', 101', 111') via the joint (41b, 51b, 61b, 71b, 81b, 91b, 101b, 111b).

5. The feedthrough according to any of the preceding claims, **characterized in that** the inner nickel pin (51', 61', 71') has a nail head.

6. The feedthrough according to any of the preceding claims, **characterized in that** the connector pin (41, 51, 61, 71, 81, 91, 101, 111) is surrounded in the flange (43, 53, 63, 73 83, 93, 103 113) by a ceramic ring (45a, 45b, 55, 65, 75, 95, 105, 115), in particular made of Al₂O₃ ceramic, and the glass solder plug (47, 57, 67, 77, 87, 97, 107, 117) is designed such that it hermetically seals an annular gap between the connector pin and the ceramic ring.

7. The feedthrough according to any of the preceding claims, **characterized in that** a filler (59, 69, 79, 89, 99, 108) surrounding the connector pin (51, 61, 71, 81, 91 101, 111) is provided inside of the glass solder plug (57, 67, 77, 87, 97, 107, 117).

8. The feedthrough according to claim 7, **characterized in that** the filler (59, 69, 79, 89, 99, 108) is dimensioned and arranged such that it masks the joint (51b, 61b, 71b, 81b) in the connector pin (51, 61, 71, 81).

9. A method for producing a feedthrough according to any of claims 1 through 8, **characterized in that** the connector pin (41, 51, 61, 71, 81 91, 101, 111) is cooled by means of a heat sink during production of the glass solder plug (57, 67, 77, 87, 97, 107, 117).

10. A use of a feedthrough according to any of claims 1 through 8, **characterized in that** interior soft soldering to the connector pin (41, 51, 61, 71, 81, 91, 101, 111) is conducted by means of a reflow method.

## Revendications

1. Conduite de passage (40, 50, 60, 70, 80, 90, 100, 110) dans un implant médical électronique avec une broche de raccordement (41, 51, 61, 71, 81, 91, 101, 111), où la broche de raccordement est une broche de niobium ou une broche en Pt, Pt/Ir, FeNi, FeNiCo, FeCr, Ta, Mo, W, Cr, FeCr, V, Ti ou leurs alliages, une bride (43, 53, 63, 73, 83, 93, 103, 113) entourant la broche de raccordement et un bouchon en verre de soudure (47, 57, 67, 77, 87, 97, 107, 117) rendant hermétiquement étanche la broche de raccordement vis-à-vis de la bride, **caractérisée en ce que** la broche de raccordement présente au moins à l'intérieur de l'implant un segment pouvant être soudé de manière douce (41', 51', 61', 71', 81', 91', 101', 111') qui comprend du nickel ou des alliages de nickel, où le segment pouvant être soudé de manière douce de la broche de raccordement dans l'intérieur de l'implant est une tige en nickel ou une tôle en nickel, où la tige en nickel ou la tôle en nickel est jointe à la broche de raccordement par le biais d'un point de jonction.

2. Conduite de passage selon la revendication 1, **caractérisée en ce que** la broche de raccordement (41, 51, 61, 71, 81, 91, 101, 111) présente au moins un noyau dans un matériau qui a approximativement le même coefficient de dilatation thermique que le produit en verre de soudure dans la partie de sa longueur dans laquelle il est en contact avec le bouchon en verre de soudure (47, 57, 67, 77, 87, 97, 107, 117).

3. Conduite de passage selon la revendication 2, **caractérisée en ce que** le matériau de la broche de raccordement (41, 51, 61, 71, 81, 91, 101, 111) ou de son noyau dans la zone de sa longueur, dans laquelle elle est en contact avec le bouchon en verre de soudure (47, 57, 67, 77, 87, 97, 107, 117) est du Pt, Pt/Ir, FeNi, FeNiCo, FeCr, Nb ou Ta.

4. Conduite de passage selon l'une des revendications précédentes, **caractérisée en ce que** la broche de raccordement est une broche en niobium, laquelle est brasée, sertie ou collée de manière électriquement conductrice sur la tige en nickel ou la tôle en nickel (41', 51', 61', 71', 81', 91', 101', 111') par le biais d'un point de jonction (41b, 51b, 61b, 71b, 81b, 91b, 101b, 111b).

5. Conduite de passage selon l'une des revendications précédentes, **caractérisée en ce que** la tige en nickel (51', 61', 71') située à l'intérieur présente une tête de clou.

6. Conduite de passage selon l'une des revendications précédentes, **caractérisée en ce que** la broche de raccordement (41, 51, 61, 71, 81, 91, 101, 111) est entourée dans la bride (43, 53, 63, 73, 83, 93, 103, 113) par un anneau en céramique (45a, 45b, 55, 65, 75, 95, 105, 115), notamment une céramique à base d'Al₂O₃, et le bouchon en verre de soudure (47, 57, 67, 77, 87, 97, 107, 117) est conçu de telle manière qu'il ferme de manière étanche hermétiquement une fente annulaire entre la broche de raccordement et l'anneau en céramique.

7. Conduite de passage selon l'une des revendications précédentes, **caractérisée en ce qu'**à l'intérieur du bouchon en verre de soudure (57, 67, 77, 87, 97, 107, 117) un produit de remplissage (59, 69, 79, 89, 99, 108) est prévu pour entourer la broche de raccordement (51, 61, 71, 81, 91, 101, 111).

8. Conduite de passage selon la revendication 7, **caractérisée en ce que** le produit de remplissage (59, 69, 79, 89, 99, 108) est dimensionné de telle manière et est disposé de sorte que le point de jonction (51b, 61b, 71b, 81b) est masqué dans la broche de raccordement (51, 61, 71, 81).

9. Procédé de fabrication d'une conduite de passage selon l'une des revendications 1 à 8, **caractérisé en ce que**, pendant la formation du bouchon en verre de soudure (47, 57, 67, 77, 87, 97, 107, 117), la broche de raccordement (41, 51, 61, 71, 81, 91, 101, 111) est refroidie au moyen d'un dissipateur de chaleur.

10. Utilisation d'une conduite de passage selon l'une des revendications 1 à 8, **caractérisée en ce que** l'on procède à une brasure douce sur le côté intérieur sur la broche de raccordement (41, 51, 61, 71, 81, 91, 101, 111) au moyen d'un procédé de brasage par refusion.
